# EUROPEAN PATENT SPECIFICATION

(11) **EP 4 048 142 B1**
(45) Date of publication and mention of the grant of the patent: **26.03.2025**
(21) Application number: 20884178.3
(22) Date of filing: 06.11.2020
(51) Int. Cl.: A61B 5/00, A61B 5/16, G16H 40/63, G16H 40/67, G16H 50/20, G16H 50/30, A61H 1/00

(54) **APPARATUS FOR MEASURING DYNAMIC VISUAL, VESTIBULAR AND SOMATOSENSORY ABILITY**
VORRICHTUNG ZUM MESSEN DER DYNAMISCHEN VISUELLEN, VESTIBULÄREN UND SOMATOSENSORISCHEN FÄHIGKEIT
APPAREIL DE MESURE DE LA CAPACITÉ VISUELLE, VESTIBULAIRE ET SOMATOSENSORIELLE DYNAMIQUE

(30) Priority: 06.11.2019 AU 2019904174; 09.06.2020 AU 2020901897
(43) Date of publication of application: 31.08.2022
(73) Proprietor: Waddington, Gordon, Australian Capital Territory ACT 2617 (AU); McGrath, Braden, Australian Capital Territory ACT 2603 (AU)
(72) Inventor: Waddington, Gordon, Australian Capital Territory ACT 2617 (AU); McGrath, Braden, Australian Capital Territory ACT 2603 (AU)
(74) Representative: Meissner Bolte Nürnberg
(86) International application number: PCT/AU2020/051205
(87) International publication number: WO 2021/087564

(56) References cited:
- WO-A1-2018/136386
- WO-A1-98/46127
- WO-A2-2004/058359
- WO-A2-2012/068040
- IT-A1- MI20 091 252
- US-A- 5 897 464
- US-A- 6 063 046
- US-A- 6 162 189
- US-A1- 2006 030 793
- US-A1- 2016 007 903
- US-A1- 2018 085 044
- US-B1- 10 231 662

## Description

### FIELD OF THE INVENTION

This present invention relates to an apparatus for making combined vestibular and somatosensory function assessment of a user of the apparatus. More particularly, but by no means exclusively, assessments made by the apparatus can be used to predict a user's athletic ability and the potential for future soft-tissue injury. Embodiments of the present invention can also be used as a rehabilitation tool for increasing vestibular and somatosensory acuity ability.

### BACKGROUND OF THE INVENTION

The somatosensory system is a part of the sensory nervous system, concerned with the conscious perception of touch, pressure, pain, temperature, position, movement, and vibration. Somatosensory ability, which includes both proprioception and tactile senses, underlies all human movement activity and can be a critical component of human motor performance.

Somatosensory ability has been shown to be: affected by injury and rehabilitation (it is worse after injury and changes with rehabilitation); indicative of falls risk in older persons (poorer somatosensory scores are associated with increased falls risk); and can be indicative of sporting performance talent (better scores in athletes are associated with better performance rankings).

The Ankle Movement Extent Discrimination Assessment (AMEDA) system was developed by one of the present inventors, Gordon Waddington, in 1995 for assessing somatosensory ability. The AMEDA system enables assessment of human somatosensory ability by assessment of the individual's ability to detect small differences in the extent of movements undertaken at the ankle without visual input. The AMEDA method has been shown to have better ecological validity and relatively better test validity and data validity than existing methods of somatosensory assessment.

While the original AMEDA does provide benefits over other existing assessment systems, it is unable to discriminate for visual and vestibular contributions of the user. It is also incapable of measuring exteroceptive inefficiency. The present inventors have found that incorporation of such factors would greatly increase the somatosensory ability assessment.

US 10 231 662 B1 discloses a force measurement system including a force measurement assembly configured to receive a subject thereon, a visual display device having an output screen, and one or more data processing devices operatively coupled to the force measurement assembly and the visual display device.

US 6 063 046 A discloses an apparatus for diagnosing balance correction abnormalities in a subject, comprising a rotatable support surface for supporting the subject and capable of rotation in pitch and roll directions, support surface control means responsive to support surface control signals for rotating the support surface in the pitch and roll directions. means for measuring a response of the subject to the rotation of the support surface and for providing response measure signals corresponding to the subject's response and system processor means for generating the support surface control signals, receiving the response measure signals, and generating an operator display from the response measure signals.

US 2006/030793 A1 discloses a device for assessing musculoskeletal dynamics of a joint of a patient in a weight-bearing posture, comprising a platform to support a patient in a weight-bearing posture, a drive mechanism to impart pulse movement to said platform. a position sensor to sense a position of said platform, a force or torque sensor to sense a force or torque on said platform and a control system to determine musculoskeletal dynamics based on outputs from said displacement sensor and said force or torque sensor.

US 6 162 189 A discloses an ankle rehabilitation system for rehabilitating an ankle of a user comprising a mobile platform adapted to be coupled to a foot of the user, a fixed platform coupled to said mobile platform, six linear actuator assemblies to determine a measured position of said mobile platform in relation to said fixed platform in six degrees of freedom each, of said linear actuator assemblies having an upper attachment end attached to said mobile platform and a lower attachment end attached to said fixed platform, means for measuring force exerted by said foot against said mobile platform in six degrees of freedom; and means for controlling said mobile platform receiving said measured position of said mobile platform and said measured force exerted by said foot against said mobile platform and applying desired force feedback in six degrees of freedom to said mobile platform.

WO 2018/136386 A1 discloses a device for assessing the balance impairment of a subject comprising a head-mounted display comprising a set of at least one display panels, an orientation sensor, a computing device, and a non-transitory computer readable medium with instructions stored thereon, that when executed by a processor, performs a method comprising presenting a sequence of scene protocols to the set of at least one display panels, recording a set of measurements from the orientation sensor and a set of time indices during the sequence of scene protocols, and calculating a balance score by referencing the set of measurements from the orientation sensors and the set of time indices to the sequence of scene protocols.

### SUMMARY OF THE INVENTION

_The invention is set forth in the independent claim 1.

### Outline of the disclosure

Embodiments result from the dependent claims and the below description.

In accordance with a first aspect there is provided an apparatus for assessing ankle movement extent discrimination of a user, comprising: a portable base unit comprising: a fixed platform; a movable platform disposed adjacent the fixed platform and being at least partially rotatable about an axis of rotation; in use, the user stands on the portable base unit in an even weight bearing stance with a first foot placed on the fixed platform and the other foot placed on the movable platform such that a long axis of the other foot is aligned with the axis of rotation of the movable platform; an adjustable stopping mechanism for adjusting an extent to which the movable platform can rotate in at least one direction with respect to a horizontal plane; and a controller for controlling the adjustable stopping mechanism to selectively adjust the extent to which the movable platform is rotated by one of a plurality of discrete predefined amounts based on a control signal; and a visual occlusion headset worn by the user, the visual occlusion headset comprising a sensing device for sensing a user response following an adjustment made by the adjustable stopping mechanism, the user response being utilised to assess ankle movement discrimination of the user.

In an embodiment the sensing device comprises one or more sensors for determining a predefined visual response effected by the user, the predetermined visual response representative of the user's recognition of the extent of the rotation effected by the adjustable stopping mechanism.

In an embodiment the headset is further configured with a display for presenting instructions to the user responsive to the adjustment made by the adjustable stopping mechanism.

In an embodiment the full visual occlusion headset further comprises a vestibular function response recording device for recording a vestibular function response of the user following an adjustment made by the adjustable stopping mechanism, or in response to a test instructed via the headset.

In an embodiment the vestibular function response recording device comprises an inclinometer and or accelerometer for recording the position of the wearers head relative to the gravitational horizontal.

In an embodiment the portable base unit further comprises an inclinometer for measuring an angle of incline of the movable platform.

In accordance with a second aspect there is provided an apparatus for making combined vestibular and somatosensory function assessments, comprising: a portable base unit comprising: a fixed platform; a movable platform being at least partially rotatable about an axis of rotation; in use, a user stands on the portable base unit in an even weight bearing stance with a first foot placed on the fixed platform and the other foot placed on the movable platform such that a long axis of the other foot is aligned with the axis of rotation for the movable platform; an adjustable stopping mechanism for adjusting an angle of rotation for the movable platform with respect to a horizontal plane; and a controller for controlling the adjustable stopping mechanism to selectively adjust the extent to which the movable platform is rotated by one of a plurality of discrete measurable amounts; a visual occlusion headset worn by the user, the headset comprising a device for recording a vestibular function response of the user associated with a vestibular function test.

In an embodiment the vestibular function response recording device comprises an inclinometer and/or an accelerometer.

In an embodiment the vestibular function test comprises presenting the user with a stimulus either through the headset or movable platform and wherein outputs of the inclinometer and/or accelerometer are utilised to determine a deviation of the user's head from true vertical.

In an embodiment the full occlusion headset comprises a display for displaying instructions and/or feedback associated with the vestibular function test.

In an embodiment the vestibular function test is associated with an adjustment made by the adjustable stopping mechanism.

In accordance with a further aspect there is provided an apparatus for making combined vestibular and somatosensory function assessments, comprising: a portable base unit comprising: a movable platform being at least partially rotatable about an axis of rotation; in use, a user stands with both feet on the movable platform; an adjustable stopping mechanism for adjusting an extent to which the movable platform can rotate in at least one direction with respect to a horizontal plane; and a controller for controlling the adjustable stopping mechanism to selectively adjust the extent to which the movable platform is rotated by one of a plurality of discrete measurable amounts based on a control signal; a visual occlusion headset worn by the user, the headset comprising a device for facilitating a vestibular function test.

In an embodiment the device comprises an inclinometer and/or an accelerometer.

In an embodiment the full occlusion headset comprises a display for displaying instructions and/or feedback associated with the vestibular function test.

In an embodiment the vestibular function test is associated with an adjustment made by the adjustable stopping mechanism.

In accordance with yet another aspect there is provided an apparatus for assessing ankle movement extent discrimination of a user, comprising: a portable base unit comprising: a fixed platform; a movable platform disposed adjacent the fixed platform and being at least partially rotatable about an axis of rotation; in use, the user stands on the portable base unit in an even weight bearing stance with a first foot placed on the fixed platform and the other foot placed on the movable platform such that a long axis of the other foot is aligned with the axis of rotation for the movable platform; an adjustable stopping mechanism for adjusting an extent to which the movable platform can rotate in a single direction with respect to a horizontal plane; and a controller for controlling the adjustable stopping mechanism to selectively adjust the extent to which the movable platform is rotated by one of a plurality of discrete measurable amounts based on a control signal for making a somatosensory assessment.

In one embodiment the apparatus further comprises a handheld device for receiving a user input and/or response associated with the vestibular function test.

In accordance with a still further aspect there is provided an apparatus for making a vestibular assessment, comprising: a portable base unit comprising: a movable platform being at least partially rotatable about an axis of rotation, in use, a user stands with both feet on the movable platform; an inclinometer coupled to the movable platform and being able to record data representative of an angle of rotation for the movable platform; a controller for receiving data output by the inclinometer and for recording a time that the user is able to maintain their balance on the movable platform without exceeding a predefined angle of rotation during an assessment period, the data received and recorded by the controller utilised for generating a vestibular response score; a visual occlusion headset worn by the user.

In an embodiment the headset comprises a display device for displaying data to the user during the assessment period for use in generating a visual response score.

In an embodiment, in one stage of the vestibular assessment, the user stands with both feet shoulder width apart and positioned directly over the rotational axis of the movable platform. In another stage of the vestibular assessment, the user stands on the movable platform with both feet shoulder width apart and astride the axis of rotation for the movable platform.

In an embodiment the apparatus further comprises a handheld device for receiving a user input and/or response related to a vestibular function assessment utilising the visual occlusion headset and wherein the user input and/or response is additionally utilised in generating the vestibular response score.

In accordance with a further aspect there is provided an apparatus for assessing ankle movement extent discrimination of a user, comprising: a base unit comprising: a fixed platform; a movable platform disposed adjacent the fixed platform and being at least partially rotatable about an axis of rotation; in use, the user stands on the portable base unit in an even weight bearing stance with a first foot placed on the fixed platform and the other foot placed on the movable platform such that a long axis of the other foot is aligned with the axis of rotation for the movable platform; an adjustable stopping mechanism for adjusting an extent to which the movable platform can rotate with respect to a horizontal plane; and a controller for controlling the adjustable stopping mechanism to selectively adjust the extent to which the movable platform is rotated; and a visual device worn by the user.

In an embodiment the apparatus further comprises a device for recording a user response following an adjustment made by the adjustable stopping mechanism, the user response comprising an indication of the adjustment sensed by the user following a rotational adjustment made to the movable platform and wherein the user response is utilised to assess ankle movement discrimination of the user. The device for recording a user response may comprise a handheld device operated by the user. The device may alternatively be incorporated into the visual occlusion device worn by the user and configured such that it senses the user response based on an eye movement made by the user.

### BRIEF DESCRIPTION OF THE ACCOMPANYING DRAWINGS

Embodiments of the present invention will now be described, by way of example only, with reference to the accompanying drawings, in which:
Figure 1 is a schematic of a system comprising a base unit, headset, and remote-control unit for implementing an embodiment of the present invention;
Figure 2 is an exploded view of the portable base unit shown in Figure 1;
Figure 3 shows a user operating the apparatus of Figure 1;
Figure 4 is an example user interface implemented by the headset of Figure 1;
Figures 5 & 6 are views illustrating various stances for performing a visual and vestibular assessment using the apparatus of Figure 4; and
Figure 7 is a schematic illustrating how visual, vestibular and somatosensory data may be combined to determine a composite performance score, in accordance with an embodiment.

### DETAILED DESCRIPTION OF EXEMPLARY EMBODIMENTS OF THE INVENTION

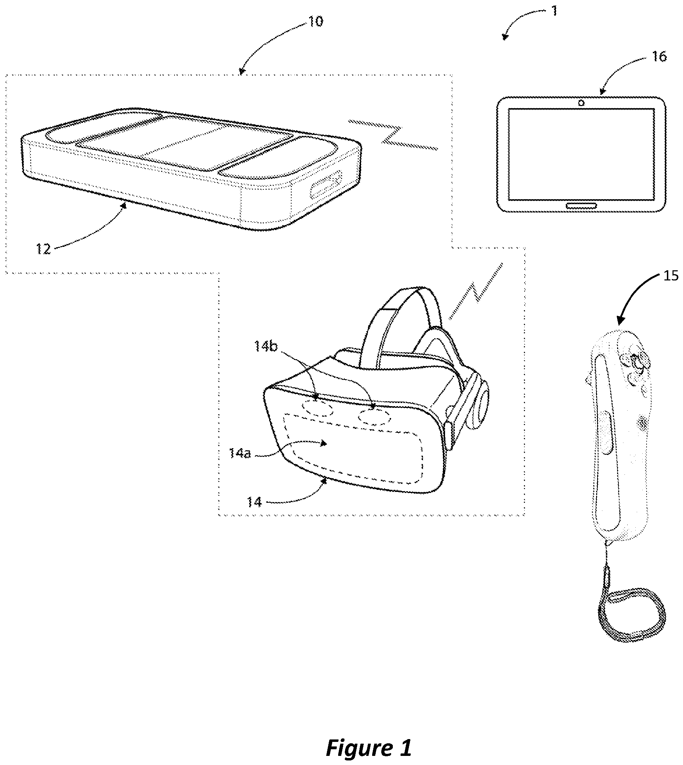

With reference to Figure 1 there is shown a schematic illustration of a system 1 implementing an apparatus 10, in accordance with an embodiment of the invention. As will be described in detail in subsequent paragraphs, the apparatus 10 enables assessment of various visual, vestibular and somatosensory parameters. Assessments made by the apparatus 10 can advantageously be used for measuring a somatosensory contribution to human motor performance ability, that in turn can be evaluated to provide an indication of a user's athletic ability, as well as their potential for injury.

According to the illustrated embodiment, the apparatus 10 comprises a base unit 12 for performing the somatosensory assessment. A headset 14 is wearable by the user for registering user responses associated with the somatosensory assessment, as well as for performing vestibular function assessments relevant to the somatosensory assessment (as will be described in more detail in subsequent paragraphs). A remote control unit 16 is provided for receiving sensor output data from the base unit 12 and headset 14, as well as for generating control signals for controlling moveable platform stopping position within the base unit 12, a display of the headset 14, etc. The remote control unit 16 may additionally be communicable with a hand-held remote 15 that allows the user to register responses to various assessments (i.e. in addition, or as an alternative to, aural responses or responses registered via the headset 14). The hand-held remote 15 may include any suitable input means for registering a user response. According to the illustrated embodiment, the remote includes depressible buttons assigned to predefined responses. According to the illustrated embodiment, the sensor data, user responses and control signals are wirelessly communicated by way of a Bluetooth connection, although it will be understood that these communications can be made using any suitable wired or wireless connection/communication protocol.

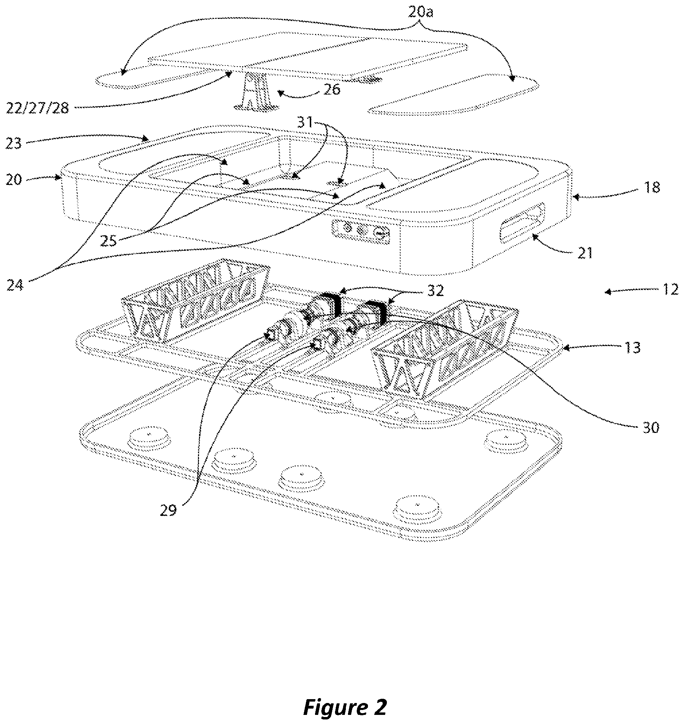

In more detail, and with additional reference to the exploded view of Figure 2, the base unit 12 comprises an elongate outer housing 18. According to the illustrated embodiment, the base unit 12 is preferably sized and shaped so as to be readily portable. Fixed platform portions 20 are located adjacent first and second ends 21, 23 of the housing 18. To facilitate grip, a non-slip pad 20a is provided in a correspondingly shaped recess defined in an upper surface of the platform portion 20 at each end 21, 23. A movable platform 22 (also referred to as a "wobble board assembly") is located adjacent in the centre of the unit 12 and has a rubberised textured upper surface that sits flush with the upper surface of the platform portion 20.

According to the illustrated embodiment, the movable platform 22 has a rectangular profile of aluminium construction, but may be comprised of any material with suitable properties. As shown, a longitudinal axis of the movable platform 22 is aligned with a longitudinal axis of the outer housing 18. The movable platform 22 is configured to seat within a recessed upper portion 24 of the outer housing 18 which has the same rectangular profile as the movable platform 22. An upper surface of the recessed portion slopes downwardly away from its midpoint (i.e. toward each end of the recess), thus creating voids 25 under corresponding portions of the movable platform 22. A gimble 26 mounted within the recessed portion 24 is centrally disposed under the movable platform 22 allowing it to pivot into either void 25 (i.e. to rotate about the platform's lateral axis in either direction).

An inclinometer 27 and accelerometer 28 are attached to, or disposed within, the movable platform 22. Readings from the inclinometer 27 and accelerometer 28 are wirelessly communicated to the remote control unit 16 by way of an electrically coupled Bluetooth module 29. It will be understood that the inclinometer output can be used to determine a relative position of the board, while the accelerometer output can be used to determine a change in velocity from one position to another.

An adjustable stopping mechanism is located in a subframe 13 of the base unit 12 for adjusting an extent to which the movable platform 22 can rotate into either void 25. Thus, a specific angle of rotation in either direction can be readily set for the platform as required for the assessment. In this instance the stopping mechanism comprises a pair of cams 30 that are mounted to the subframe 13 and which are each rotatable into and out of a corresponding void 25 through an opening 31. Each cam 30 is coupled to a servo motor assembly 32 for effecting its rotation. The cams 30 are rotatable to allow various discrete stopping points to be set for the movable platform 22, thus providing a suite of predefined ankle movement ranges when the movable platform is rotated from the horizontal (as will be described in more detail in subsequent paragraphs). The servo motor assemblies 32 are electrically connected to the Bluetooth module 29 for receiving wireless control signals from the remote control unit 16. In this regard, the remote control unit 16 is controlled to issue a control signal for selectively adjusting the rotation of either cam 30 to achieve a desired stop position for the movable platform 22. As will be described in subsequent paragraphs, the desired stop position can be manually entered via the remote control unit 16, or automatically selected based on a control sequence.

Returning to Figure 1, the headset 14 worn by the user takes the form of a visual occlusion headset comprising a visual display 14a and sensing device 14b for sensing a user response following a rotational adjustment of the movable platform 22. According to one embodiment, the sensing device comprises an eye tracker 14b having one or more sensors for determining a predefined visual response effected by the user (i.e. which is representative of the user's recognition of the movable board position). For example, the user might direct their gaze to a particular point on the display screen which registers a predefined movable board angle with respect to the horizontal (e.g. 1 degree, 2 degrees, 3 degrees, etc.). As another example, the user may to look to a particular point of the display screen 14a to indicate a lesser or greater angle of adjustment with respect to the previous adjustment. In a particular embodiment, the remote control unit 16 may be programmed with a computer program that iteratively creates a sequence of stop positions in response to the number of correct answers supplied by a user to enable dynamic movement sequences to be generated, thus optimising somatosensory testing functionality. In yet another alternative embodiment, which is not part of the invention, the headset 14 may serve primarily to block out external stimulus, in which case the user may simply verbalise the adjustment/angle that they have sensed, which verbalised output is then recorded (either manually by the assessor or automatically by speech recognition software and hardware implemented by the headset or other suitable apparatus). As yet another alternative, which is also not part of the invention, the user may register their response via the hand-held remote 15. The headset 14 may additionally be configured with an inclinometer and/or accelerometer for recording a position of the user's head relative to the gravitational horizontal.

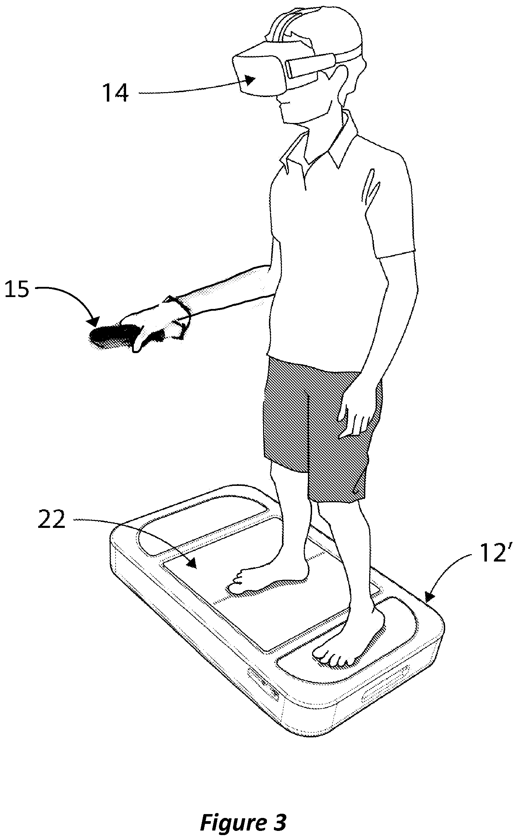

With additional reference to Figure 3, a basic somatosensory assessment involves the user standing on the portable base unit 12 in an even weight bearing stance, with one foot placed on one of the fixed platforms 20 and the other foot placed on the midline of the movable platform 22 such that a long axis of each foot is aligned with the lateral axis of the movable platform 22. The cams 30 can be controlled so as to set a maximum angle of rotation for the movable platform 22 in either direction. It will be understood that the cams 30 may be controlled so that the movable platform 22 can only rotate in a single direction, depending on the particular assessment being carried out. The user recorded outputs (recorded either aurally, via the headset 14, or the hand-held remote 15) are then evaluated against the actual board angles for generating a somatosensory score. The somatosensory score may, for example, be a percentage score representative of how closely the user recorded outputs were to the actuals. Alternatively, the somatosensory score may be determined from a participant's discrimination between each pair of adjacent movement extents calculated using signal detection analysis generating areas under the response curves. It will be understood that various somatosensory ability scores may be derived and applied, depending on the desired implementation.

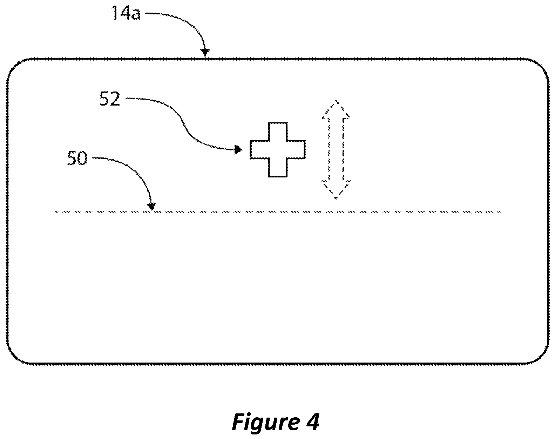

The contribution of visual function to upright stance can additionally be assessed by having the user maintain a cursor within a field of the headset display 14a, aligned as closely as possible to a fixed target line displayed in the centre of the headset display field. An example field of display is shown in Figure 4, where the target line is denoted by reference numeral 50 and the curser denoted by reference numeral 52. The headset 14 is programmed such that the relative position of the cursor 52 within the headset display field is linked simultaneously to the position of the inclinometer 27 attached to the movable platform 22 of the portable base unit 12. The headset background display and target line 50 is referenced to the horizontal of the surface of the base unit 12.

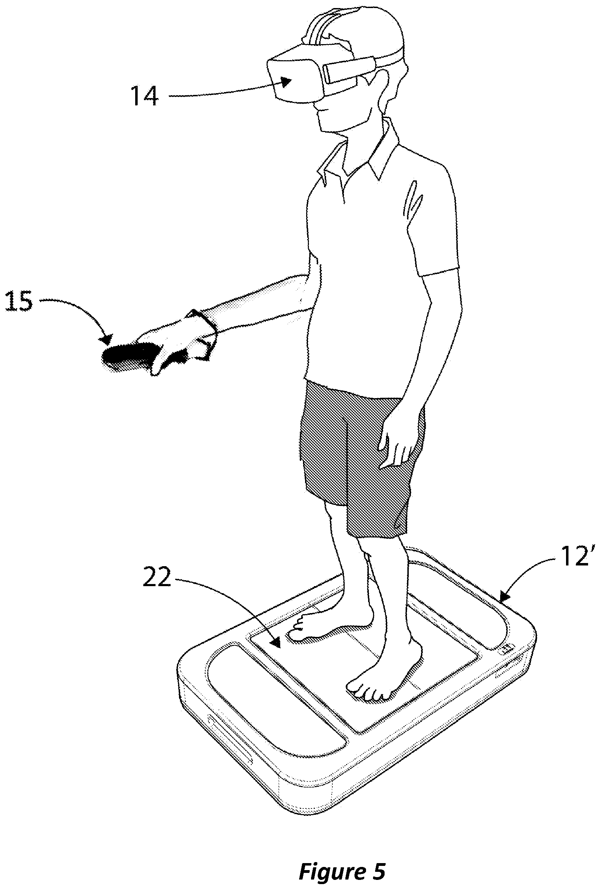

A form of combined vestibular, somatosensory assessment is performed in two stages. In a first stage, for anterior-posterior assessment, the user stands on the movable platform 22 with both feet shoulder width apart and positioned directly over the axis of rotation of the movable platform 22 (i.e. such the long axis of each foot is aligned perpendicular to the axis of rotation of the movable platform, in this instance the lateral axis). This is best shown in Figure 5. In this position the user's body is undertaking a pitch movement. Once suitably positioned, the user is required to balance on the platform 22 to maintain its position as close to horizontal as possible, within predefined limits, for some predefined time, e.g. 20 seconds. The remote control unit 16 records a stability metric for the first assessment. In this example, the stability metric is the time that the user was able to maintain the movable platform 22 within 2.5 degrees of the horizontal (determined based on data output from the inclinometer 27). Data output from the accelerometer may also be recorded and evaluated.

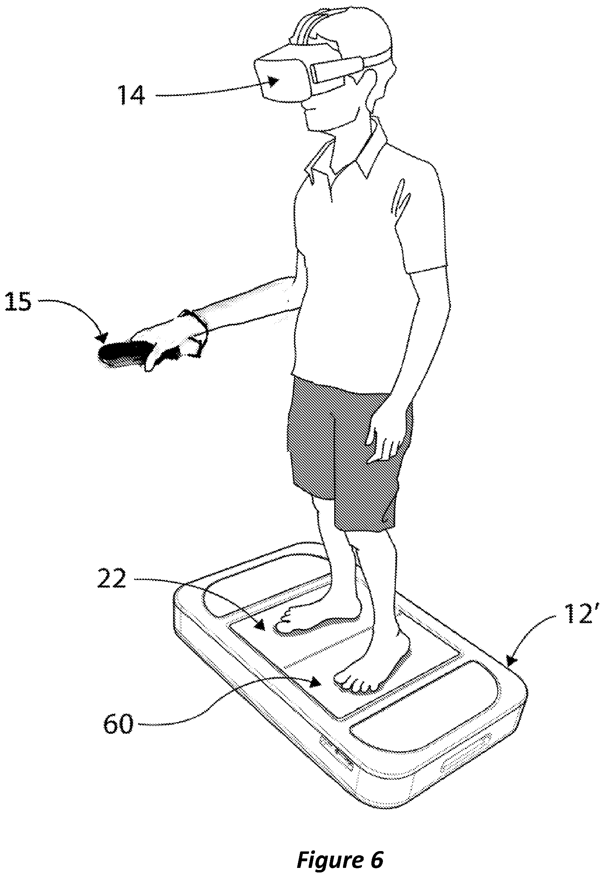

In a second assessment stage, the user is instructed to stand on the movable platform 22 with both feet shoulder width apart and astride the axis of rotation such that their feet are aligned with the axis of rotation of the movable platform 22 (see Figure 6). In this position the user's body is undertaking a roll movement. The remote control unit 16 then records the same stability metric as for the first stage (i.e. the time the user is able to maintain the movable platform within 2.5 degrees of horizontal). Again, acceleration data may be recorded for the second stage. The remote control unit 16 then determines an average stability measure for the two stages (using the data output from the inclinometer and accelerometer) and provides a feedback score against a normative data set. It will be understood that the feedback score may be based on only one of the stage assessment metrics, although this may result in a less meaningful output.

The headset 14 is configured to measure a form of vestibular function. In this regard, the sensor device 14b comprises InfraRed (IR) eye tracking cameras within the headset which are calibrated to each user. Calibration software may, for example, involve the user looking at a green dot in four key directions; right, up, left and down. By way of example, one form of vestibular function may be assessed by making eye gaze evaluations, examples of which are described below. In this assessment the user is asked to keep their head still and to look straight ahead. The assessment begins with a dark background displayed on the headset display 14a. During the assessment, a moving target is displayed on the headset display 14a and the movement of the subject's pupils in response to a change in the location of the target is recorded by the IR cameras 14b and communicated to the remote control unit 16 for subsequent evaluation.

In a particular embodiment, the headset 14 may also be configured to measure an additional form of vestibular function. In this regard the sensor device comprises an inclinometer and/or accelerometer coupled to the headset 14 which measures the position of the user's head. The user is presented with a stimulus which could be visual through the headset display, or somatosensory through the platform, and asked to maintain their head in an upright position. The deviation of the head position from true vertical is measured and communicated to the remote control unit 16 for subsequent evaluation.

In an additional vestibular function assessment, the hand-held remote 15 may be utilised for recording a user input associated with a test presented via the headset 14. For example, the user may be asked to use the remote 15 to orient a line shown in the headset display so that it aligns with earth horizontal. For a combined vestibular somatosensory assessment, the user may be asked to use the hand-held remote 15 to orient a line shown in the headset display to be equal to the position of the moveable platform 22.

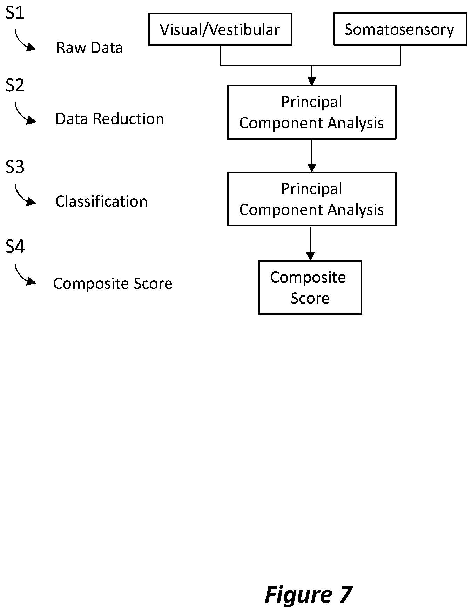

The visual, vestibular and somatosensory assessment data output using one or more of the above assessments may be combined to create a single Composite Performance Score (CPS), using a data reduction process as schematically illustrated in Figure 7.

Combining and presenting this raw data in a single score may provide an easily interpretable measure avoiding the potential for information overload. It will be understood that the score may be calculated by the remote control unit, or some other suitable computer processing arrangement.

In more detail, at Step S1, the visual, vestibular and somatosensory data is gathered and input to a data reduction process, where the data gets condensed (step S2). By way of example, a Principal Component Analysis (PCA) may be used to reduce the raw input data. As persons skilled in the art will understand, PCA is a technique used to identify patterns in data, by mapping the raw data into a lower dimensional space. The resultant reduced data is provided as the input to a Support Vector Machine (SVM) machine learning classifier which, at step S4, calculates the CPS.

Using a headset 14 to record user response has several notable advantages. Firstly, the headset 14 occludes the user's vision, thereby preventing external stimulus that can adversely impact on the assessment. The headset 14 also allows the user to stand upright during the somatosensory assessment and discourages the user from looking at the portable base unit 12, which again can adversely impact on assessment. The headset 14 also provides an efficient and speedy interface for recording the user response.

It will be understood that the remote control unit 16 as described above could take the form of any suitable computing arrangement with the appropriate software and hardware for communicating with the portable base unit 12 and headset 14. For example, the remote control unit may take the form of a computer tablet, laptop computer or the like. In another form, the remote control unit 16 may be directly coupled or incorporated into any one of the portable unit 12, handheld remote 15 or headset 14. It will also be understood that the unit 12 may be configured with a fixed platform on only one side of the unit, without departing from the scope of the invention.

Embodiments of the present disclosure may be described in the general context of computer-executable instructions, such as program components or modules, executed by one or more computers or other devices. Aspects of the present disclosure may be implemented with any number and organisation of components or modules.

A computer or controller, such as those described herein, includes at least one processor or processing unit and a system memory. The computer or controller typically has at least some form of computer readable media. By way of example and not limitation, computer readable media include computer storage media and communication media. Computer storage media include volatile and non-volatile, removable and non-removable media implemented in any method or technology for storage of information such as computer readable instructions, data structures, program modules, or other data. Communication media typically embody computer readable instructions, data structures, program modules, or other data in a modulated data signal such as a carrier wave or other transport mechanism and include any information delivery media. Those skilled in the art are familiar with the modulated data signal, which has one or more of its characteristics set or changed in such a manner as to encode information in the signal. Combinations of any of the above are also included within the scope of computer readable media.

In the claims which follow and in the preceding description of the invention, except where the context requires otherwise due to express language or necessary implication, the word "comprise" or variations such as "comprises" or "comprising" is used in an inclusive sense, i.e. to specify the presence of the stated features but not to preclude the presence or addition of further features in various embodiments of the invention.

## Claims

1. An apparatus (10) for making combined vestibular and somatosensory function assessments of a user of the apparatus, comprising:
a portable base unit (12) comprising:
a fixed platform (20);
a rotatable platform (22) being at least partially rotatable freely about a horizontal axis of rotation without the aid of any external drive;
said rotation being carried out only by the force of the ankle movement of a user standing on said platform (22);
in use, a user stands on the portable base unit (12) in an even weight bearing stance with a first foot placed on the fixed platform (20) and the other foot placed on the rotatable platform (22) such that a long axis of the other foot is aligned with the axis of rotation for the rotatable platform (22);
an adjustable stopping mechanism configured to adjust a maximal angle of rotation for the rotatable platform (22) with respect to a horizontal plane to which the rotatable platform (22) can be rotated by the user of the apparatus (10); and
a controller (16) configured to control various discrete stopping positions of the rotatable platform (22) by controlling the adjustable stopping mechanism to selectively adjust the extent to which the rotatable platform (22) can be rotated by the user by one of a plurality of discrete measurable amounts;
a visual occlusion headset (14) worn by the user, the headset (14) comprising a device configured to record a vestibular function response of the user associated with a vestibular function test, wherein the visual occlusion headset (14) comprises a sensing device (14b) configured to sense a user response following an adjustment made by the adjustable stopping mechanism, and wherein the sensing device (14b comprises an eye tracker (14b) having one or more sensors configured to determine a predefined visual response effected by the user, the predefined visual response being representative of the user's perception of the extent of the rotation of the rotatable platform (22) following an adjustment made by the adjustable stopping mechanism,
wherein the controller (16) is further configured to receive the predefined visual response from the visual occlusion headset (14) and to utilise it to perform ankle movement extent discrimination assessment of the user as a somatosensory function assessment.

2. An apparatus (10) in accordance with claim 1, wherein the visual occlusion headset (14) is further configured with a display (14a) for presenting instructions to the user responsive to the adjustment made by the adjustable stopping mechanism.

3. An apparatus (10) in accordance with claim 2, wherein the vestibular function response recording device comprises an inclinometer (27) and/or an accelerometer (28) for recording the position of the wearers head relative to the gravitational horizontal.

4. An apparatus (10) in accordance with claim 3, further comprising a device for recording a user response following an adjustment made by the adjustable stopping mechanism, the user response comprising an indication of the extent of the rotatable platform (22) range of motion limited by the adjustment as perceived by the user following a rotational adjustment made to the rotatable platform (22) and wherein the user response is utilised to assess ankle movement discrimination of the user and wherein the device is either a handheld device (15) operated by the user or a device incorporated into the visual occlusion device (14) and is configured to sense the user response based on an eye movement made by the user.

5. An apparatus (10) in accordance with claim 4, wherein the vestibular function test comprises presenting the user with a stimulus either through the headset (15) or movable platform and wherein outputs of the inclinometer (27) and/or accelerometer (28) are utilised to determine a deviation of the user's head from true vertical.

6. An apparatus (10) in accordance with claim 5, wherein the visual occlusion headset (15) comprises a display (14a) for displaying instructions and/or feedback associated with the vestibular function test and wherein the vestibular function test is associated with an adjustment made by the adjustable stopping mechanism.

## Patentansprüche

1. Vorrichtung (10) zur Durchführung kombinierter vestibulärer und somatosensorischer Funktionsbewertungen eines Benutzers der Vorrichtung, umfassend:
eine tragbare Basiseinheit (12), die Folgendes umfasst:
eine feste Plattform (20);
eine drehbare Plattform (22), die zumindest teilweise frei um eine horizontale Drehachse ohne Hilfe eines externen Antriebs drehbar ist;
wobei diese Drehung nur durch die Kraft der Knöchelbewegung eines auf der Plattform (22) stehenden Benutzers ausgeführt wird;
im Gebrauch steht ein Benutzer auf der tragbaren Basiseinheit (12) in einer gleichmäßig belasteten Haltung, wobei ein erster Fuß auf der festen Plattform (20) und der andere Fuß auf der drehbaren Plattform (22) platziert ist, so dass eine Längsachse des anderen Fußes mit der Drehachse der drehbaren Plattform (22) ausgerichtet ist;
einen einstellbaren Stoppmechanismus, der so konfiguriert ist, dass er einen Drehwinkel für die drehbare Plattform (22) in Bezug auf eine Horizontale einstellt, gegenüber der die drehbare Plattform (22) durch den Benutzer der Vorrichtung (10) gedreht werden kann; und
eine Steuereinrichtung (16), die so konfiguriert ist, dass sie verschiedene diskrete Haltepositionen der drehbaren Plattform (22) steuert, indem sie den einstellbaren Stoppmechanismus steuert, um das Ausmaß, in dem die drehbare Plattform (22) durch den Benutzer gedreht werden kann, selektiv um einen von mehreren diskreten messbaren Beträgen einzustellen;
ein visuelles Okklusions-Headset (14), das von dem Benutzer getragen wird, wobei das Headset (14) eine Einrichtung umfasst, die so konfiguriert ist, dass sie eine vestibuläre Funktionsreaktion des Benutzers aufzeichnet, die mit einem vestibulären Funktionstest assoziiert ist, wobei das visuelle Okklusions-Headset (14) eine Erfassungsvorrichtung (14b) umfasst, die so konfiguriert ist, dass sie eine Benutzerreaktion nach einer durch den einstellbaren Stoppmechanismus vorgenommenen Einstellung erfasst, und wobei die Erfassungsvorrichtung (14b) einen Eye-Tracker umfasst, der einen oder mehrere Sensoren aufweist, die so konfiguriert sind, dass sie eine vordefinierte visuelle Reaktion bestimmen, die von dem Benutzer bewirkt wird, wobei die vordefinierte visuelle Reaktion repräsentativ für die Wahrnehmung des Benutzers des Ausmaßes der Drehung der drehbaren Plattform (22) nach einer durch den einstellbaren Stoppmechanismus vorgenommenen Einstellung ist, wobei die Steuereinrichtung (16) ferner so konfiguriert ist, dass sie die vordefinierte visuelle Reaktion von dem visuellen Okklusions-Headset (14) empfängt und verwendet, um eine Bewertung des Ausmaßes der Knöchelbewegung des Benutzers als eine somatosensorische Funktionsbewertung durchzuführen.

2. Vorrichtung (10) nach Anspruch 1, wobei das visuelle Okklusions-Headset (14) ferner mit einem Display (14a) konfiguriert ist, um dem Benutzer als Reaktion auf die durch den einstellbaren Stoppmechanismus vorgenommene Einstellung Anweisungen zu präsentieren.

3. Vorrichtung (10) nach Anspruch 2, wobei die Vorrichtung zur Aufzeichnung der vestibulären Funktionsreaktion einen Neigungsmesser (27) und/oder einen Beschleunigungsmesser (28) zur Aufzeichnung der Position des Kopfes des Trägers relativ zur Schwerkrafthorizontalen umfasst.

4. Vorrichtung (10) nach Anspruch 3, ferner umfassend eine Einrichtung zum Aufzeichnen einer Benutzerreaktion nach einer durch den einstellbaren Stoppmechanismus vorgenommenen Einstellung, wobei die Benutzerreaktion eine Anzeige des Ausmaßes des durch die Einstellung begrenzten Bewegungsbereichs der drehbaren Plattform (22) umfasst, wie sie vom Benutzer nach einer an der drehbaren Plattform (22) vorgenommenen Dreheinstellung wahrgenommen wird, und wobei die Benutzerreaktion verwendet wird, um die Knöchelbewegungsunterscheidung des Benutzers zu bewerten, und wobei die Einrichtung entweder eine vom Benutzer betriebene Handvorrichtung (15) oder eine in dem visuellen Okklusions-Headset (14) eingebaute Einrichtung ist und so konfiguriert ist, dass sie die Benutzerreaktion auf der Grundlage einer vom Benutzer ausgeführten Augenbewegung erfasst.

5. Vorrichtung (10) nach Anspruch 4, wobei der vestibuläre Funktionstest umfasst, dass dem Benutzer ein Reiz entweder durch das Headset (15) oder die bewegliche Plattform dargeboten wird und wobei die Ausgaben des Neigungsmessers (27) und/oder des Beschleunigungsmessers (28) verwendet werden, um eine Abweichung des Kopfes des Benutzers von der wahren Vertikalen zu bestimmen.

6. Vorrichtung (10) nach Anspruch 5, wobei das visuelle Okklusions-Headset (15) ein Display (14a) zur Anzeige von Anweisungen und/oder Rückmeldungen umfasst, die mit dem vestibulären Funktionstest verbunden sind, und wobei der vestibuläre Funktionstest mit einer Einstellung verbunden ist, die durch den einstellbaren Stoppmechanismus vorgenommen wird.

## Revendications

1. Appareil (10) permettant d'effectuer des évaluations combinées des fonctions vestibulaires et somatosensorielles d'un utilisateur de l'appareil, comprenant:
une unité de base portable (12) comprenant
une plate-forme fixe (20);
une plate-forme rotative (22) pouvant au moins partiellement tourner librement autour d'un axe de rotation horizontal sans l'aide d'un entraînement externe;
cette rotation est effectuée uniquement par la force du mouvement de la cheville d'un utilisateur se tenant sur ladite plate-forme (22 ;
lors de l'utilisation, l'utilisateur se tient debout sur l'unité de base portable (12) dans une position d'équilibre avec un premier pied placé sur la plate-forme fixe (20) et l'autre pied placé sur la plate-forme rotative (22) de telle sorte que l'axe long de l'autre pied est aligné avec l'axe de rotation de la plate-forme rotative (22);
un mécanisme d'arrêt réglable configuré pour régler un angle de rotation de la plate-forme rotative (22) par rapport à l'horizontale à laquelle la plate-forme rotative (22) peut être tournée par l'utilisateur de l'appareil (10); et
un contrôleur (16) configuré pour contrôler diverses positions d'arrêt discrètes de la plate-forme rotative (22) en contrôlant le mécanisme d'arrêt réglable pour ajuster sélectivement la mesure dans laquelle la plate-forme rotative (22) peut être tournée par l'utilisateur par l'une d'une pluralité de quantités discrètes mesurables;
un casque d'occlusion visuelle (14) porté par l'utilisateur, le casque (14) comprenant un dispositif configuré pour enregistrer une réponse de la fonction vestibulaire de l'utilisateur associée à un test de la fonction vestibulaire, dans lequel le casque d'occlusion visuelle (14) comprend un dispositif de détection (14b) configuré pour détecter une réponse de l'utilisateur à la suite d'un ajustement effectué par le mécanisme d'arrêt réglable, et dans lequel le dispositif de détection (14b) comprend un traqueur oculaire ayant un ou plusieurs capteurs configurés pour déterminer une réponse visuelle prédéfinie effectuée par l'utilisateur, la réponse visuelle prédéfinie est représentative de la perception par l'utilisateur de l'étendue de la rotation de la plate-forme rotative (22) à la suite d'un ajustement effectué par le mécanisme d'arrêt réglable, le contrôleur (16) étant en outre configuré pour recevoir la réponse visuelle prédéfinie du casque d'occlusion visuelle (14) et l'utiliser pour effectuer une évaluation de la discrimination de l'étendue du mouvement de la cheville de l'utilisateur en tant qu'évaluation de la fonction somatosensorielle.

2. Appareil (10) selon la revendication 1, dans lequel le casque d'occlusion visuelle (14) est en outre configuré avec un écran (14a) pour présenter des instructions à l'utilisateur en réponse à l'ajustement effectué par le mécanisme d'arrêt réglable.

3. Appareil (10) selon la revendication 2, dans lequel le dispositif d'enregistrement de la réponse de la fonction vestibulaire comprend un inclinomètre (27) et/ou un accéléromètre (28) pour enregistrer la position de la tête du porteur par rapport à l'horizontale gravitationnelle.

4. Appareil (10) selon la revendication 3, comprenant en outre un dispositif pour enregistrer une réponse de l'utilisateur à la suite d'un ajustement effectué par le mécanisme d'arrêt réglable, la réponse de l'utilisateur comprend une indication de l'étendue de l'amplitude de mouvement de la plate-forme rotative (22) limitée par l'ajustement, telle que perçue par l'utilisateur à la suite d'un ajustement rotatif effectué sur la plate-forme rotative (22) et dans laquelle la réponse de l'utilisateur est utilisée pour évaluer la discrimination du mouvement de la cheville de l'utilisateur et dans laquelle le dispositif est soit un dispositif portable (15) actionné par l'utilisateur, soit un dispositif incorporé dans le dispositif d'occlusion visuelle (14) et est configuré pour détecter la réponse de l'utilisateur sur la base d'un mouvement oculaire effectué par l'utilisateur.

5. Appareil (10) selon la revendication 4, dans lequel le test de la fonction vestibulaire consiste à présenter à l'utilisateur un stimulus par l'intermédiaire du casque (15) ou de la plate-forme mobile et dans lequel les sorties de l'inclinomètre (27) et/ou de l'accéléromètre (28) sont utilisées pour déterminer une déviation de la tête de l'utilisateur par rapport à la verticale réelle.

6. Appareil (10) selon la revendication 5, dans lequel le casque d'occlusion visuelle (15) comprend un écran (14a) pour afficher des instructions et/ou un retour d'information associés au test de la fonction vestibulaire et dans lequel le test de la fonction vestibulaire est associé à un réglage effectué par le mécanisme d'arrêt réglable.
